# EUROPEAN PATENT APPLICATION

(11) **EP 4 279 062 A1**
(43) Date of publication of application: **22.11.2023**
(21) Application number: 23173612.5
(22) Date of filing: 16.05.2023
(51) Int. Cl.: A61K 9/00, A61K 47/10, A61K 47/26, A61K 31/137, A61P 25/00

(54) **LIQUID COMPOSITION AND ITS USE, TREATMENT METHOD AND KIT**

(30) Priority: 17.05.2022 BR 102022009562
(71) Applicant: EMS S.A., CEP-13186-901 Hortolandia, SP (BR)
(72) Inventor: LOUZA DE OLIVEIRA, Rodrigo, 13186-901 Sao Paulo (BR); SANTOS, Fernando Henrique, 13186-901 Sao Paulo (BR); RIBEIRO CATELLI, Ettamyr Eduardo, 13186-901 Sao Paulo (BR)
(74) Representative: Oetke, Cornelia

(57) **Abstract**

The present invention discloses new liquid compositions of lisdexamfetamine, their uses, kits and treatment methods for treating attention deficit hyperactivity disorder (ADHD) and binge eating disorder (BED).

## Description

### TECHNICAL FIELD

The present invention belongs to the pharmaceutical field and relates to liquid compositions of lisdexamfetamine or a pharmaceutically acceptable salt thereof, its uses, kits and treatment methods for treating attention deficit hyperactivity disorder (ADHD) and binge eating disorder (BED).

### FUNDAMENTALS OF THE INVENTION

Lisdexamfetamine (LDX) or L-lysine-d-amphetamine, whose IUPAC nomenclature is (2S)-2,6-diamino-N-[(1S)-1-methyl-2-phenylethyl]hexanamide, is a psychostimulant medicament and received approval from the United States Food and Drug Administration (FDA) for the treatment of Attention Deficit Hyperactivity Disorder (ADHD) in adults in April 2008 and is currently indicated for the treatment of ADHD also in children and teenagers, and for the treatment of Binge Eating Disorder (BED) in adults only.

Lisdexamfetamine dimesylate is a white to off-white powder, very soluble in water and soluble in methanol, melting point 192-198°C, molecular formula C₁₇H₃₃N₃O₇S₂ and molecular weight 455.59 g/mol.

Lisdexamfetamine is a prodrug of dextroamphetamine and contains D-amphetamine covalently linked to the essential amino acid L-lysine. After oral administration, lisdexamphetamine is rapidly absorbed from the gastrointestinal tract and hydrolyzed primarily in blood cells to dextroamphetamine, which is responsible for the drug's activity. Amphetamines are non-catecholamine sympathomimetic amines with stimulant activity of the central nervous system.

As the first psychostimulant prodrug, lisdexamfetamine represents the class of long-acting agents for the treatment of ADHD. LDX has a biological delivery mechanism that uses enzymatic hydrolysis to convert the inactive molecule into the active drug, d-amphetamine.

Although the mechanism of action of drugs indicated for the treatment of ADHD is unknown, their effectiveness is probably related to the increase in synaptic concentrations of catecholamines. Both methylphenidate and amphetamine, the two types of stimulants commonly used to treat ADHD, are believed to increase the efflux and function of norepinephrine and dopamine in the central nervous system.

Attention Deficit Hyperactivity Disorder (ADHD) is a neuropsychiatric condition that can affect both children and adults. In children, symptoms can manifest as significant social, emotional, and academic problems.

Binge Eating Disorder (BED) is characterized by the ingestion, in a period of two hours, of a larger amount of food than other people would consume under similar circumstances. During bingeing episodes, the individual eats faster than normal and feelings of shame and guilt are reported due to the amount of food ingested.

Several drugs have been studied for the treatment of BED, such as dasotraline, lisdexamfetamine, methylphenidate, armodafinil, oxycytone, naltrexone + bupropion, vortioxetine, liraglutide, disulfiram, among others. However, currently, only lisdexamfetamine is approved by the FDA and ANVISA for the treatment of BED.

There are solid compositions of lisdexamfetamine on the market, in particular in the form of capsules. For example, Venvanse^{®} 30 mg, manufactured by Shire, is in a capsule format, where each capsule contains 30 mg of lisdexamfetamine dimesylate equivalent to 17.34 mg of lisdexamfetamine.

However, the pharmaceutical formulation in solid form presents problems related to administration, dosage and adherence to treatment.

For example, people with difficulty swallowing medications may experience problems when treating with a composition in the form of capsules. In these cases, there was the possibility of opening the hard capsules to have their contents dissolved in pasty foods, such as yogurt or in a glass of water or orange juice. However, this alternative has disadvantages in terms of practicality and the possibility of wasting and losing part of the capsule's drug content.

As of the present disclosure, there are no liquid lisdexamfetamine compositions. Thus, it is urgent to develop a pharmaceutical composition of lisdexamfetamine in liquid form, which has good administration and dosage characteristics, in addition to good stability and solubility, and which also favors adherence to treatment.

The document PI0612440-2 describes compounds comprising a chemical moiety covalently linked to amphetamine to reduce or prevent amphetamine abuse and overdose. In said document, compositions of lisdexamfetamine useful for the treatment of attention deficit disorder are disclosed. Among the various embodiments exemplified in said document, no liquid or syrup formulation containing lisdexamfetamine is disclosed.

Indeed, there are a number of challenges related to the development of liquid base formulations of lisdexamfetamine, such as its stability and solubility. Specifically, it is known that active pharmaceutical ingredients in liquid form are more susceptible to physical and chemical instability than in solid form.

In this sense, for the case of oral solutions, the active pharmaceutical ingredient needs to be chemically and physically stable. Specifically, trace amounts of impurities in active pharmaceutical ingredients or excipients and the pH of the solution can cause the active pharmaceutical ingredient to degrade, increasing solution instability.

Thus, although acid addition salts of lisdexamfetamine, such as lisdexamphetamine dimesylate and lisdexamfetamine hydrochloride, are very soluble in water, interest in developing an oral liquid solution of a salt of lisdexamfetamine is hampered by the fact that the molecule degrades significantly under basic (alkaline) and oxidative conditions.

In this context, it is advantageous to develop new alternative compositions of lisdexamfetamine that can be more easily administered, with dosage accuracy, greater practicality, allowing adherence to treatment, titration security, ease of homogenization and that have suitable pharmacological action, in addition to high stability and solubility, providing good shelf-life characteristics.

Therefore, the present invention relates to new and inventive liquid compositions of lisdexamfetamine or a pharmaceutically acceptable salt thereof.

### SUMMARY OF THE INVENTION

The present invention discloses new lisdexamfetamine liquid compositions, their uses, kits and treatment methods to treat attention deficit hyperactivity disorder (ADHD) and binge eating disorder (BED).

The innovative composition can be administered to a patient in need of treating attention deficit hyperactivity disorder (ADHD) and binge eating disorder (BED), ensuring accuracy and speed of dosing, in addition to enabling adequate adherence to treatment.

A preferred embodiment of the invention refers to a liquid composition of lisdexamfetamine, associated with pharmaceutically acceptable excipients and vehicles.

In another embodiment of the invention, the use of said liquid composition to prepare a medicament to treat attention deficit hyperactivity disorder (ADHD) and binge eating disorder (BED) is described.

Another preferred embodiment of the invention relates to the method of treating attention deficit hyperactivity disorder (ADHD) and binge eating disorder (BED).

A further embodiment of the invention refers to a kit comprising the liquid composition of lisdexamfetamine associated with a suitable delivery device and, optionally, administration instructions.

Surprisingly, the inventors of the present application developed a liquid pharmaceutical composition of lisdexamfetamine, which maintained the pharmacological action of the compositions of lisdexamfetamine in solid form already existing in the state of the art, while providing greater comfort and adherence of the patient to the treatment, in addition to precise control of the dosage and solubility of the composition, without the adverse effects of instability resulting from the degradation of lisdexamfetamine in alkaline or oxidative conditions.

### DETAILED DESCRIPTION OF THE INVENTION

The present invention relates to compositions comprising the pharmaceutically active ingredient lisdexamfetamine dimesylate, which has the following structural formula:

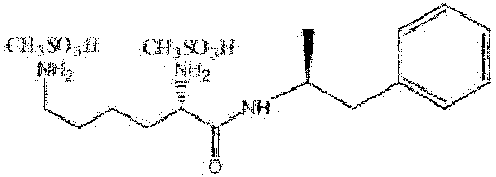

As indicated herein, the prior art already presents lisdexamfetamine compositions. However, it fails to disclose or suggest the necessary teachings to achieve the main object of the present invention, namely, a liquid composition of lisdexamfetamine or a pharmaceutically acceptable salt thereof with a small amount of water and antioxidant to prevent degradation by oxidation of lisdexamfetamine and to help the preservation of the composition.

In particular, despite the known significant degradability of lisdexamfetamine under alkaline and oxidative conditions, the inventors of the present invention were unexpectedly able to obtain a new pharmaceutical form of a composition of lisdexamfetamine dimesylate, with low amount of water and antioxidant, which assists in maintaining good characteristics of solubility and stability of the active ingredient during and after the preparation process of the liquid composition.

When compared to the pharmaceutical form in capsules, the liquid composition has the advantage of being easier to use, through improved administration and ingestion. Consequently, the liquid pharmaceutical form provides greater comfort and adherence of patients in the treatment of attention deficit hyperactivity disorder (ADHD) and binge eating disorder (BED), especially those with difficulty swallowing capsules.

Additionally, the liquid composition allows precise control of the dosage of the active pharmaceutical ingredient, without the adverse effects of instability arising from the degradation of lisdexamfetamine under alkaline or oxidative conditions.

In a first embodiment, the liquid composition of the present invention comprises lisdexamfetamine dimesylate and water, associated with pharmaceutically acceptable excipients and vehicles. Preferably the liquid composition of the present invention is in the form of a drop solution or syrup.

According to the present invention, pharmaceutically acceptable excipient is defined as any substance, other than the active pharmaceutical ingredient, which has been evaluated for its safety and/or is approved for pharmaceutical use by a competent regulatory agency, if applicable.

Such excipients are selected according to the pharmaceutical dosage form of interest, its route of administration, physical-chemical compatibility with the active ingredient and the effect on efficacy. For example, pharmaceutically acceptable excipients can be selected from ROWE, R.C. et al. "Handbook of Pharmaceutical Excipients", 6th ed. USA, 2009, the Brazilian Pharmacopoeia or foreign correspondent, such as the American Pharmacopoeia or the European Pharmacopoeia.

According to the present invention, the one or more pharmaceutically acceptable excipients can be selected from the group consisting of preservatives, sweeteners, thickeners, stabilizers, flavoring agents, antioxidants, pH adjusting agents, chelators, solvents, solubilizers, vehicles, wherein the same excipient may have more than one function in the composition. In some embodiments of the invention, the composition comprises as excipients solvents, antioxidants, sweeteners, flavoring agents, preservatives, pH adjusting agents and vehicles.

According to the present invention, the main solvent can be selected from the group consisting of mono-, di-, or trihydroxy alcohols, pharmaceutically acceptable solvents, or combinations thereof. In preferred embodiments, the solvent is selected from the group consisting of glycerol, propylene glycol, sorbitol or combinations thereof.

According to the present invention, water can be used as a co-solvent, i.e., as a secondary solvent.

The liquid composition has antioxidants that help the preservation thereof. According to the present invention, the antioxidants can be selected from the group consisting of butylhydroxyanisole (BHA), butylhydroxytoluene (BHT) and tocopherol. Preferably, the antioxidant used in the present invention is butylhydroxyanisole (BHA).

According to the present invention, the sweetener can be selected from the group consisting of natural or synthetic sweeteners, or combinations thereof, which impart sweet taste to the composition. In preferred embodiments, the sweetener is selected from the group consisting of neohesperidin dihydrochalcone, steviol glycosides, mannitol, sorbitol, sucralose, thaumatin, xylitol, and combinations thereof. In even more preferred embodiments, the sweetener is sucralose.

According to the present invention, the flavoring agent can be selected from the group consisting of any agents capable of masking unpleasant tastes present in the composition, including natural and artificial flavorings. In preferred embodiments, the flavoring is selected from the group consisting of strawberry flavor, cherry essence, raspberry essence, orange essence, mint essence, strawberry essence, chocolate essence and combinations thereof. In even more preferred embodiments, the flavoring agent is strawberry flavor.

According to the present invention, the preservative may be selected from the group consisting of phenoxyethanol, methylisothiazolinone, methylchloroisothiazolinone, methylparaben, propylparaben and combinations thereof. In preferred embodiments, the preservative is methylparaben.

According to the present invention, the pH adjusting agent can be selected from the group consisting of sodium hydroxide, citric acid and combinations thereof. In preferred embodiments, the pH adjusting agent is sodium hydroxide.

According to the present invention, the vehicle can be selected from the group consisting of purified water, esters in general, mineral oils, vegetable oils and mixtures thereof. In preferred embodiments, the vehicle is purified water.

During the development of new compositions, the solubilization and stabilization of lisdexamfetamine dimesylate in liquid medium proved to be challenging considering the degradability of lisdexamfetamine under alkaline or oxidative conditions, allowing the appearance of impurities and, thus, impairing the stability of the product.

Thus, the inventors prepared several combinations in order to identify optimized concentration ranges for the components of the composition, namely, lisdexamfetamine dimesylate associated with one or more excipients and vehicles. Such preliminary tests indicated that the complete exclusion of water was detrimental to maintaining the flavor of the composition.

In some embodiments of the invention, the final concentration of lisdexamfetamine dimesylate in the composition can range from 20 to 60 mg/ml of the composition. In preferred embodiments, the final concentration of lisdexamfetamine dimesylate in the composition can range from 30 to 50 mg/ml of the composition. In even more preferred embodiments, the final concentration of lisdexamfetamine dimesylate in the composition is 40 mg/ml of the composition.

In some embodiments of the invention, the final concentration of water in the liquid composition can range from 5 to 25% w/w based on the total weight of the composition. In preferred embodiments, the final water concentration in the liquid composition can range from 10 to 20% w/w based on the total weight of the composition. In further preferred embodiments, the final concentration of water in the liquid composition is 15% w/w based on the total weight of the composition.

In some embodiments of the invention, the total concentration of solvents in the liquid composition can range from 65 to 85% w/w based on the total weight of the composition. In preferred embodiments, the total concentration of solvents in the liquid composition can range from 70 to 80% w/w based on the total weight of the composition. In further preferred embodiments, the total concentration of solvents in the liquid composition is 75% w/w based on the total weight of the composition.

As previously mentioned, until the moment of the present disclosure, no pharmaceutical composition for lisdexamfetamine dimesylate in the administration form and with the concentration disclosed herein had been taught or even suggested. In this sense, the inventors of the present application developed new pharmaceutical forms of lisdexamfetamine dimesylate that maintained its pharmacological action, while allowing suitable patient comfort and adherence, as well as precise control of the dosage and solubility of the composition, without the adverse effects of instability resulting from the degradation of the lisdexamfetamine under alkaline or oxidative conditions.

In another embodiment, the present invention presents the use of lisdexamfetamine dimesylate liquid composition to prepare a medicament to treat attention deficit hyperactivity disorder (ADHD) and binge eating disorder (BED) in a patient. Preferably, the patient is human.

In a further embodiment, the present invention features a method of treating attention deficit hyperactivity disorder (ADHD) and binge eating disorder (BED) by administering a therapeutically effective amount of the liquid composition of dimesylate lisdexamfetamine to a patient in need thereof. Preferably, the patient is human.

Further, in some preferred embodiments, the administration of the liquid composition of lisdexamfetamine dimesylate is orally.

The liquid composition of lisdexamfetamine dimesylate of the present invention can be prepared through a process comprising the following steps:
a) in an auxiliary reactor, add propylene glycol and methylparaben under stirring;
b) manually transfer the product resulting from step a) to the main reactor;
c) in an auxiliary reactor, add sorbitol syrup (70%), butylhydroxyanisole (BHA) and glycerin under stirring and heating;
d) transfer the product resulting from step c) to the main reactor;
e) add purified water, glycerin, sorbitol syrup (70%) and sodium hydroxide to the main reactor;
f) add lisdexamfetamine under stirring to the main reactor;
g) add sucralose and strawberry flavor (SC542045) under agitation to the main reactor;
h) add purified water under stirring to the main reactor until a homogeneous, colorless solution with strawberry flavor and aroma is obtained;
i) filter the product obtained in step h) in the main reactor and transfer to a reservoir tank;
j) pass the resulting solution through a primary packaging/filling step in a filling machine; and
k) perform the volume control by weight and check the closure of the flasks.

In another embodiment, the present invention presents a kit comprising the disclosed liquid composition of lisdexamfetamine dimesylate. The kit according to the present invention may comprise such a composition associated with any suitable delivery device and, optionally, instructions for administration. Delivery devices commonly used in the pharmaceutical field include, without limitation, drippers, ampoules, syringes and others.

### EXAMPLES

The examples below, described in detail, serve to illustrate embodiments of the present invention without having, however, a limiting character to the scope of protection thereof.

### Example 1 - Process of preparing a liquid composition of lisdexamfetamine dimesylate

The following is a process for preparing a liquid composition of lisdexamfetamine dimesylate.

In an auxiliary reactor, propylene glycol and methylparaben are added under stirring, at a temperature between 15-30°C, with a stirring speed of 260 rpm and a homogenization time of 30 minutes. Afterwards, the resulting product is manually transferred to the main reactor.

In an auxiliary reactor, sorbitol syrup (70%), butylhydroxyanisole (BHA) and glycerin are added under stirring and heating, at a temperature between 70-80°C, with a stirring speed of 260 rpm. Afterwards, the resulting product is transferred to the main reactor under a temperature of 30-80°C, with a stirring speed of 1000 rpm and a homogenization time of 10 minutes.

Purified water, glycerin, sorbitol syrup (70%) and sodium hydroxide are added to the main reactor at a temperature between 15-30°C, with a stirring speed of 1200 rpm.

Then, the active pharmaceutical ingredient lisdexamfetamine is added under stirring to the main reactor, with stirring speed of 1200 rpm.

Subsequently, sucralose and strawberry flavoring (SC542045) are added under stirring to the main reactor, with stirring speed of 1200 rpm.

Purified water is then added under agitation to the main reactor, with stirring speed of 1200 rpm until a homogeneous, colorless solution with strawberry flavor and odor, and a pH between 5.0-7.0 is obtained.

The product obtained above in the main reactor is filtered and transferred to a reservoir tank, for a blowing time of 600 seconds, with transfer pressure (nitrogen type) of 800 mB, wherein the filter porosity is 40 microns.

Then, the resulting solution goes through a primary packaging/filling step in a filling machine, with the aid of a torque wrench from 4 to 25 inlb, in order to obtain the following volumes: minimum: 50.00 mL; medium: 50.50 mL; and maximum: 51.00 mL.

Finally, a volume control by weight is performed and the closure of the bottles is checked.

### Example 2 - Exemplary liquid composition

The following is an exemplary liquid composition of lisdexamfetamine dimesylate prepared in accordance with the present invention. Table 1 indicates each of the raw materials, amounts, proportions and respective functions in the exemplary formulation.

**Table 1: Exemplary liquid composition**

| **Feedstock** | **Quantity (mg/ml)** | **% w/w in the pharmaceutical form** | **Function in the formula** |
|---|---|---|---|
| Lisdexamfetamine (A3) | 40.000 | 4.000 | Active principle |
| Propylene glycol | 200.000 | 20.000 | Solvent |
| Methylparaben | 1.500 | 0.150 | Preservative |
| Butylhydroxyanisole (BHA) | 0.100 | 0.010 | Antioxidant |
| Sucralose | 15.000 | 1.500 | Sweetener |
| Glycerin | 200.000 | 20.000 | Solvent |
| Sorbitol syrup (70%) | 350.000 | 35.000 | Solvent |
| Sodium hydroxide | 0.100 | 0.010 | pH corrector |
| Strawberry Flavor (SC542045) | 4.000 | 0.400 | Flavoring |
| Purified water | 155.818 | 15.582 | Vehicle |
| Glycerin q.s.p. | QSP 1 mL | 3.348 (qsp 100) | Solvent |
| Total | 1.000 mL | 100% | |

### Example 3 - Stability Test Results

After completing the development of the liquid composition of lisdexamfetamine dimesylate 40 mg/ml solution drops, according to Example 2, three pilot batches named LP155/2021, LP156/2021 and LP157/2021 were manipulated. The three pilot batches were submitted to a stability study and showed satisfactory results (at 30°C/75%RH and 40°C/75%RH).

It should be understood that the embodiments described above are merely illustrative and that various modifications can be made by a person skilled in the art to the same without departing from the scope of the present invention. Consequently, the present invention should not be considered limited to the exemplary embodiments described in the present application. Furthermore, the present disclosure may include subject matter not currently claimed, but which can be claimed in the future in combination with or separately from the features claimed herein.

## Claims

1. A liquid composition **characterized in that** the composition comprises lisdexamfetamine dimesylate at a concentration from 20 to 60 mg/ml associated with pharmaceutically acceptable excipients and vehicles, wherein the liquid composition has an amount of water in the range from 5 to 25% w/w based on the total weight of the composition.

2. The liquid composition according to claim 1, **characterized in that** the lisdexamfetamine dimesylate is present at a concentration from 30 to 50 mg/mL.

3. The liquid composition according to claim 1 or 2, **characterized in that** the lisdexamfetamine dimesylate is present at a concentration of 40 mg/mL.

4. The liquid composition according to any one of claims 1 to 3, **characterized in that** the water is present in an amount in the range from 10 to 20% w/w based on the total weight of the composition.

5. The liquid composition according to any one of claims 1 to 4, **characterized in that** the water is present in an amount of 15% w/w based on the total weight of the composition.

6. The liquid composition according to any one of claims 1 to 5, **characterized in that the** total solvent concentration ranges from 65 to 85% w/w based on the total weight of the composition.

7. The liquid composition according to any one of claims 1 to 6, **characterized in that** the total solvent concentration varies from 70 to 80% w/w based on the total weight of the composition.

8. The liquid composition according to any one of claims 1 to 7, **characterized in that** the total solvent concentration is 75% w/w based on the total weight of the composition.

9. The liquid composition according to any one of claims 1 to 8, **characterized in that** the composition is in the form of a solution.

10. The liquid composition according to claim 9, **characterized in that** the composition is in the form of a drop solution or a syrup.

11. The liquid composition according to any one of claims 1 to 10, **characterized in that** the composition is administered orally.

12. A use of the liquid composition of any one of claims 1 to 11, **characterized in that** it is for preparing a medicament to treat attention deficit hyperactivity disorder (ADHD) and binge eating disorder (BED).

13. A method for treating attention deficit hyperactivity disorder (ADHD) and binge eating disorder (BED) **characterized in that** it is through administering to a patient in need thereof a therapeutically effective amount of the liquid composition as defined in any one of claims 1 to 11.

14. A kit **characterized in that** the kit comprises the liquid composition as defined in any one of claims 1 to 11, a delivery device and, optionally, administration instructions.

15. The kit according to claim 14, **characterized in that** the delivery device is a calibrated dripper for standardizing the volume of the liquid formulation.
